(19) Europäisches Patentamt
European Patent Office
Office européen des brevets

(11) Veröffentlichungsnummer : **0 010 515**
**B1**

# (12) EUROPÄISCHE PATENTSCHRIFT

(45) Veröffentlichungstag der Patentschrift : **17.10.84**

(51) Int. Cl.$^3$ : **C 07 D307/86**, A 01 N 47/34

(21) Anmeldenummer : **79810075.6**

(22) Anmeldetag : **27.08.79**

(54) **(N-Methylcarbamoyl-(2,2,-dimethyl-(2H,3H)-dihydrobenzofuran-7-yloxy)-(N'.N".N"-trialkyl-harnstoff)-N.N'-sulfidderivate, Verfahren zu ihrer Herstellung, Mittel, welche diese Derivate als aktive Komponente enthalten und deren Verwendung zur Bekämpfung von In.**

(30) Priorität : **31.08.78 CH 9197/78**
**12.03.79 CH 2328/79**
**07.06.79 CH 5309/79**

(43) Veröffentlichungstag der Anmeldung : **30.04.80 Patentblatt 80/09**

(45) Bekanntmachung des Hinweises auf die Patenterteilung : **17.10.84 Patentblatt 84/42**

(84) Benannte Vertragsstaaten : **AT BE CH DE FR GB IT NL**

(56) Entgegenhaltungen :
**DE-A- 2 045 440**
**US-A- 3 925 056**
**B. Wegler: "Chemie der Pflanzenschutz und Schädlingsbekämpfungsmittel" Bd. 1 (1970), Springer Berlin, S. 231, 234, 235, 237**

(73) Patentinhaber : **CIBA-GEIGY AG**
**Postfach**
**CH-4002 Basel (CH)**

(72) Erfinder : **Drabek, Jozef, Dr.**
**Benkenstrasse 12**
**CH-4104 Oberwil (CH)**
Erfinder : **Böger, Manfred**
**Lindenstrasse 33**
**CH-7858 Weil am Rhein 5 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft neue [N-Methylcarbamoyl-(2,2-dimethyl-(2H,3H)-dihydrobenzofuran-7-yloxy]-(N',N'',N''-trialkyl-harnstoff)-N,N'-sulfidderivate, welche eine Wirkung gegen Schädlinge, vor allem gegen Insekten besitzen, und Verfahren zu ihrer Herstellung sowie insektizide Mittel, welche die vorstehend genannten Derivate als aktive Komponente enthalten, und Verfahren zur Bekämpfung von Insekten unter Verwendung der neuen Verbindungen.

Aus der DOS Nr. 2 045 440 ist die Verbindung der Formel

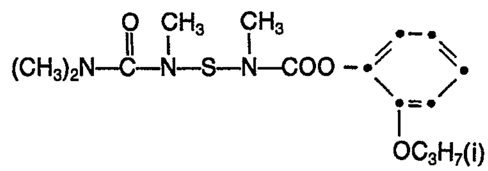

als Insektizid bereits bekannt.

Demgegenüber haben die neuartigen, insektiziden, erfindungsgemässe Verbindungen überraschenderweise eine bedeutend stärkere Kontaktwirkung, gegen Myzus persicae.

Die erfindungsgemässen neuen [N-Methylcarbamoyl-(2,2-dimethyl-(2H,3H)-dihydrobenzofuran-7-yloxy)-(N',N'',N''-trialkyl-harnstoff)-N,N'-sulfidderivate entsprechen der Formel I

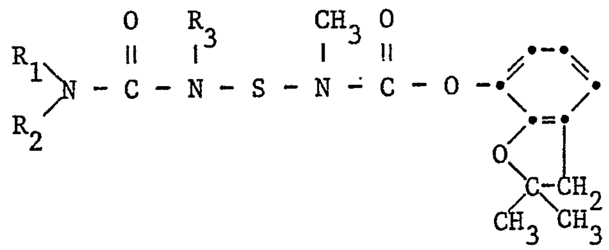

(I)

worin $R_1$ und $R_2$ je eine $C_1$-$C_4$-Alkylgruppe und $R_3$ eine $C_1$-$C_{10}$-Alkyl- oder $C_3$-$C_6$-Cycloalkylgruppe bedeuten.

Alkylgruppen als $R_1$ und $R_2$, die gleich oder verschieden sind, sowie als $R_3$ können verzweigt oder geradkettig sein. Als Beispiele solcher Gruppen kommen die Methyl-, Aethyl-, n- und i-Propyl-, n-, i-, s- und t-Butylgruppe sowie die n-Pentyl, n-Hexyl-n-Octyl- und n-Decylgruppe in Betracht. In den Verbindungen der Formel I werden die folgenden Substituententypen sowie Kombinationen derselben untereinander bevorzugt:

1) Bei $R_1$ und $R_2$: Methyl oder Aethyl;
2) Bei $R_3$: $C_1$-$C_8$-Alkyl (insbesondere $C_1$-$C_4$-Alkyl und vor allem Methyl) und Cyclopropyl.

Es hat sich nun erfindungsgemäss überraschenderweise gezeigt, dass die erwähnten Verbindungen der Formel I eine vorzügliche insektizide Wirkung mit breitem Spektrum aufweisen. So können sie z. B. zur Bekämpfung von Insekten der Ordnungen Lepidoptera, Coleoptera, Heteroptera, Diptera, Orthoptera und Homoptera eingesetzt werden.

In diesem Zusammenhang ist hervorzuheben, dass die Verbindungen der Formel I sich durch eine besonders stark ausgeprägte Aktivität gegen Insekten der zuletzt genannten Ordnung (Homoptera), und vor allem gegen Insekten der Familie Aphididae auszeichnen. Dabei wurde beispielsweise festgestellt, dass die erfindungsgemässen Verbindungen sowohl eine Kontakt- als auch eine systemische Wirkung gegen eine grössere Anzahl von Vertretern der genannten Familie (wie z. B. Aphis fabae, Aphis craccivora und Myzus persicae), welche sich mit anderen Mitteln nur schwierig bekämpfen lassen, besitzen.

Diesen Eigenschaften zufolge sind die Verbindungen der Formel I erfindungsgemäss zur Bekämpfung von Insekten, vor allem pflanzenschädigenden Insekten, in Kulturen von Nutz- und Zierpflanzen, hauptsächlich auf dem Gebiet des Gemüse-, Obst- und Zitrusbaues, besonders geeignet.

Darüber hinaus besitzen die Verbindungen der Formel I eine wertvolle Wirkung gegen pflanzenparasitäre Nematoden sowie gegen Akariden, insbesondere ektoparasitäre Akariden (Milben und Zecken), z. B. der Familien Ixodidae, Argasidae und Dermanyssidae.

Erfindungsgemäss wurde weiter festgestellt, dass die vorstehend beschriebenen pestiziden Eigenschaften mit einer für die praktische Anwendung auf dem Gebiet des Pflanzenschutzes vorteilhaften Toxizität gegenüber Warmblütern gekoppelt sind.

Die Verbindungen der Formel I werden analog bekannten Verfahren hergestellt, indem man z. B.

a) eine Verbindung der Formel II

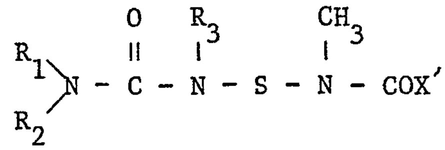

(II)

in Gegenwart einer Base mit einer Verbindung der Formel III

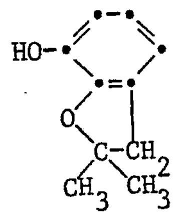

(III)

umsetzt ; oder

b) eine Verbindung der Formel IV

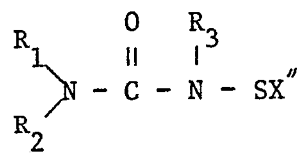

(IV)

in Gegenwart einer Base mit einer Verbindung der Formel V

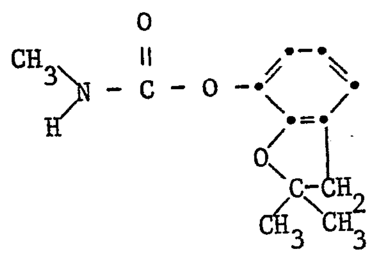

(V)

reagieren lässt ; wobei in den Formeln II, III, IV und V $R_1$ bis $R^3$ die für Formel I bereits angegebenen Bedeutungen haben und X′ für ein Halogen- insbesondere Fluoratom und X″ für ein Halogen- insbesondere Chloratom stehen.

Die Verfahren (a) und (b) werden bei einer Reaktionstemperatur zwischen − 50 °C und + 130 °C, vorzugsweise zwischen − 10 °C und + 100 °C, bei normalem oder leicht erhöhtem Druck und in Gegenwart eines gegenüber den Reaktionsteilnehmern inerten Lösungs- oder Verdünnungsmittels vorgenommen.

Als für diese Verfahren geeignete Basen kommen insbesondere tertiäre Amine, wie Trialkylamine, Pyridine und Dialkylaniline ferner Hydroxide, Oxide, Carbonate und Bicarbonate von Alkali- und Erdalkalimetallen sowie Alkalimetallalkoholate wie z. B. Kalium-tert. butylat und Natriummethylat in Betracht.

Als Lösungs- oder Verdünnungsmittel eignen sich z. B. Aether und ätherartige Verbindungen wie Diäthyläther, Di-iso-propyläther, Dioxan, Tetrahydrofuran ; aliphatische und aromatische Kohlenwasserstoffe, insbesondere Benzol, Toluol, Xylole ; und Ketone wie Aceton, Methyläthylketon und Cyclohexanon.

Die in dem vorstehend aufgeführten Verfahren verwendeten Ausgangsstoffe der Formel III, II, IV und V sind bekannt bzw. können analog zu den bekannten Methoden hergestellt werden (siehe z. B. GB Patent Nr. 1,138,347 ; US Patent Nr. 3,474,171 und 3,111,539 ; DOS 1,910,588 und DOS 2,045,440).

Die Ausgangsstoffe der Formel II werden aus bekannten Vorläufern erhalten, indem man z. B. eine Verbindung der Formel VI

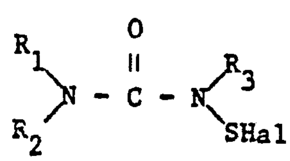

(VI)

in Gegenwart einer Base mit einer Verbindung der Formel VII

$$CH_3—NH—COX' \quad (VII)$$

umsetzt, wobei in den Formeln VI und VII $R_1$, $R_2$, $R_3$ und X' die bereits erwähnten Bedeutungen haben und « Hal » für ein Halogenatom steht.

Das Verfahren zur Herstellung der Ausgangsstoffe der Formel II wird vorzugsweise in Gegenwart eines gegenüber den Reaktionsteilnehmern inerten Lösungs- oder Verdünnungsmittels, bei einer Reaktionstemperatur von −50 °C bis +130 °C, unter normalem Druck durchgeführt. Als für dieses Verfahren geeignete Basen und Lösungsmittel kommen die für die oben aufgeführten Verfahren (a) und (b) bereits erwähnten Substanzen in Betracht.

Die Verbindungen der Formel I werden erfindungsgemäss als solche verwendet oder bilden einen Bestandteil von Mitteln, welche noch geeignete Trägerstoffe oder Zuschlagstoffe oder Gemische solcher Stoffe enthalten.

Geeignete Träger- und Zuschlagstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs-, Binde- und/oder Düngemitteln.

Die insektizide Wirkung der erfindungsgemässen Mittel lässt sich durch Zusatz anderer Akarizide und/oder Insektizide wesentlich verbreitern.

Als Zusätze eignen sich z. B. : org. Phosphorverbindungen ; Nitrophenole und deren Derivate ; Fornamidine ; Harnstoffe ; pyrethrinartige Verbindungen ; Karbamate und chlorierte Kohlenwasserstoffe.

Die erfindungsgemässen Mittel können z. B. als Stäubemittel, Granulate, Dispersionen, Lösungen und Aufschlämmungen sowie als in Wasser dispergierbare Spritzpulver, Pasten, Emulsionen und Emulsionskonzentrate vorliegen und angewendet werden.

Der Gehalt an Wirkstoff (Verbindung der Formel I) in den oben beschriebenen Mitteln liegt zwischen 0,1 bis 95 %, dabei ist zu erwähnen, dass bei der Applikation aus dem Flugzeug oder mittels anderer geeigneter Applikationsgeräte auch höhere Konzentrationen eingesetzt werden können.

Die Verbindungen der Formel I können beispielsweise wie folgt formuliert werden :

### Emulsionskonzentrat I

20 Gew.-Teile des Wirkstoffes werden in
70 Gew.-Teilen Xylol gelöst und mit
10 Gew.-Teilen eines Emulgiermittels, bestehend aus einem Gemisch eines Arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

### Emulsionskonzentrat II

5 bis max. 30 Gew.-Teile Wirkstoff werden unter Rühren bei Zimmertemperatur in
30 Gew.-Teilen Dibutylphthalat
10 Gew.-Teilen Solvent 200 (niederviskoses hocharomat. Erdöldestillat)
15 bis 35 Gew.-Teilen Dutrex 238 FC (viskoses hocharomat. Erdöldestillat) gelöst und mit
10 Gew.-Teilen eines Emulgatorgemisches, bestehend aus Rizinusölpolyglykoläther und dem Calciumsalz der Docecylbenzolsulfonsäure, versetzt. Das so erhaltene Emulsionskonzentrat gibt in Wasser milchige Emulsionen.

### Spritzpulver

5 bis 30 Gew.-Teile des Wirkstoffes werden in einer Mischapparatur mit
5 Gew.-Teilen eines aufsaugenden Trägermaterials (Kieselsäure K 320 oder Wessalon S) und
55 bis 80 Gew.-Teilen eines Trägermaterials (Bolus alba oder Kaolin B 24) und einem Dispergiermittelgemisch, bestehend aus
5 Gew.-Teilen eines Na-lauryl-sulfonates und
5 Gew.-Teilen eines Alkyl-aryl-polyglykoläthers, intensiv vermischt. Diese Mischung wird auf einer Stift- oder Luftstrahlmühle bis auf 5-15 µm gemahlen. Das so erhaltene Spritzpulver gibt in Wasser eine gute Suspension.

### Stäubemittel

5 Gew.-Teile feingemahlener Wirkstoff werden mit
2 Gew.-Teilen einer gefällten Kieselsäure und
93 Gew.-Teilen Talk intensiv gemischt.

Pour-on-Lösung

| | |
|---|---|
| Wirksubstanz | 30,0 g |
| Natrium-dioctylsulfosuccinat | 3,0 g |
| Benzylalkohol | 48,0 g |
| Erdnussöl | 19,8 g |
| | 100,8 g = 100 ml |

Die Wirksubstanz wird in dem Benzylalkohol unter Rühren, eventuell auch unter leichtem Erwärmen, gelöst. Zu der Lösung wird das Natrium-dioctylsulfosuccinat und das Erdnussöl gegeben und unter Erwärmen und gründlichem Durchmischen gelöst.

Die nachfolgenden Beispiele dienen zur näheren Erläuterung der Erfindung.

## Beispiel 1

a) Herstellung von (N-Methylcarbamoylfluorid)-(N',N'',N''-trimethylharnstoff)-N,N'-sulfid (Ausgangsstoff).

Zu einer Lösung von 5 g wasserfreier Fluorwasserstoffsäure in 5 ml Toluol wurden bei einer Temperatur von − 50 °C unter Rühren 14,85 ml Methylisocyanat zugegeben und das Reaktionsgemisch wurde während 2 Stunden nachgerührt. Zu der auf diese Weise erhaltenen Lösung wurden 42,1 g N,N,N'-Trimethylharnstoffsulfonylchlorid zugegeben und bei einer Temperatur von − 50° bis − 20 °C unter Rühren 34,55 ml Triäthylamin zugetropft. Danach wurde das Reaktionsgemisch während 2 Stunden bei − 20 °C und anschliessend während 10 Stunden bei Raumtemperatur gerührt. Nach dem Abnutschen wurde das Filtrat am Rotationsdampfer eingeengt. Nach dem Abdestillieren des Rohproduktes am Hochvakuum erhielt man das (N-Methylcarbamoylfluorid)-(N',N'',N''-trimethylharnstoff)-N,N'-sulfid der Formel

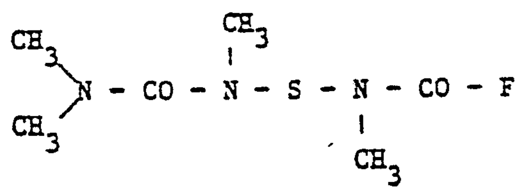

als orange Flüssigkeit mit einem Kp. von 78-83 °C bei 0,04 mb.

Die folgenden Ausgangsstoffe der Formel IIa

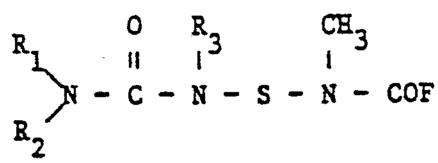

(IIa)

sind auf analoge Weise erhältlich :

(Siehe Tabelle Seite 6 f.)

| $R_1$ | $R_2$ | $R_3$ | Physikalische Daten |
|---|---|---|---|
| $CH_3$ | $CH_3$ | 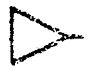 | Kp.: 100-105°C / 0,09 mb |
| $CH_3$ | $CH_3$ | $n-C_4H_9$ | Kp.: 105-110°C / 0,08 mb |
| $CH_3$ | $CH_3$ | $n-C_6H_{13}$ | Kp.: 125-127°C / 0,09 mb |
| $CH_3$ | $CH_3$ | $n-C_8H_{17}$ | Kp.: 140-145°C / 0,13 mb |
| $CH_3$ | $CH_3$ | $n-C_{10}H_{21}$ | |
| $C_2H_5$ | $C_2H_5$ | $CH_3$ | Kp.: 97-99°C / 0,11 mb |
| $C_2H_5$ | $C_2H_5$ | $n-C_4H_9$ | Kp: 120-121°C / 0,08 mb |
| $nC_3H_7$ | $nC_3H_7$ | $CH_3$ | Kp.: 109-111°C / 0,07 mb |
| $iC_3H_7$ | $iC_3H_7$ | $CH_3$ | Kp.: 96-103°C / 0,11-0,2 mb |
| $nC_4H_9$ | $CH_3$ | $n-C_4H_9$ | Kp.: 125-128°C / 0,07 mb |
| $n-C_4H_9$ | $n-C_4H_9$ | $CH_3$ | Kp: 119-124°/ 0,08 mb |
| $n-C_4H_9$ | $n-C_4H_9$ | $n-C_4H_9$ | Kp: 135-136°/ 0,08 mb |
| $CH_3$ | $CH_3$ | 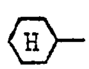 | |

b) Herstellung von (N-Methylcarbamoyloxy-(2,2-dimethyl-(2H,3H)-dihydrobenzofuran-7-yl))-(N',N",N"-trimethyl-harnstoff)-N,N'-sulfid (Endprodukt).

Zu einer Lösung von 8,75 g 2,2-Dimethyl-(2H,3H)-dihydro-7-hydroxy-benzofuran in 80 ml Toluol wurden unter Rühren 7,4 ml Triäthylamin und anschliessend bei max. 30 °C 11,15 g (N-Methylcarbamoyl-fluorid)-(N',N",N", trimethyl-harnstoff)-N,N'-sulfid zugetropft. Das Reaktionsgemisch wurde 16 Std. bei Raumtemperatur und 2 1/2 Std. bei 45 °C nachgerührt, filtriert und eingeengt. Das Rohprodukt wurde in Benzol aufgenommen, 4 x mit je 50 ml Wasser gewaschen und die organische Phase über Natriumsulfat getrocknet und anschliessend eingeengt. Auf diese Weise erhielt man das (N-Methylcarbamoyloxy-(2,2-dimethyl-(2H,3H)-dihydrobenzofuran-7-yl))-N',N"-N"-trimethylharnstoff)-N,N'-sulfid der Formel

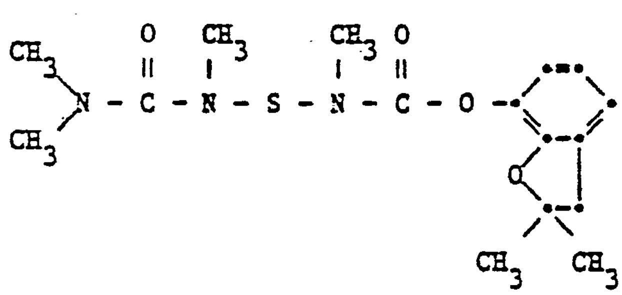

(Verbindung Nr. 1) als gelbes Oel mit einem Brechungsindex von $n_D^{45}$ : 1,530 8.

Die nachfolgenden Verbindungen der Formel IA, IB und IC können analog zu dem vorstehend beschriebenen Herstellungsverfahren hergestellt werden :

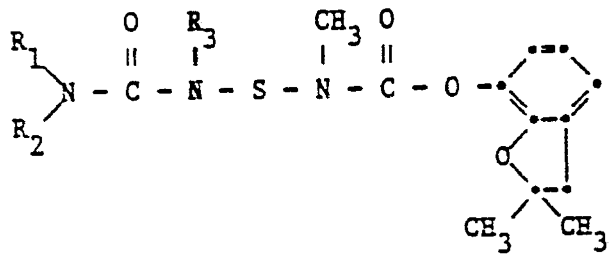

(IA)

| Verb.Nr. | $R_1$ | $R_2$ | $R_3$ | Physikalische Daten |
|---|---|---|---|---|
| 2 | $CH_3$ | $CH_3$ | 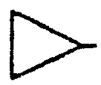 | $n_D^{45}$ : 1,5329 |
| 3 | $CH_3$ | $CH_3$ | $n-C_4H_9$ | |
| 4 | $CH_3$ | $CH_3$ | $n-C_6H_{13}$ | $n_D^{30}$ : 1,5119 |
| 5 | $CH_3$ | $CH_3$ | $n-C_8H_{17}$ | $n_D^{30}$ : 1,5060 |
| 6 | $CH_3$ | $CH_3$ | $n-C_{10}H_{21}$ | |
| 7 | $C_2H_5$ | $C_2H_5$ | $CH_3$ | $n_D^{40}$ : 1,5239 |
| 8 | $C_2H_5$ | $C_2H_5$ | $n-C_4H_9$ | $n_D^{40}$ : 1,5109 |
| 9 | $n-C_3H_7$ | $n-C_3H_7$ | $CH_3$ | $n_D^{40}$ : 1,5160 |
| 10 | $i-C_3H_7$ | $i-C_3H_7$ | $CH_3$ | $n_D^{40}$ : 1,5170 |
| 11 | $n-C_4H_9$ | $CH_3$ | $n-C_4H_9$ | $n_D^{40}$ : 1,5040 |
| 12 | $n-C_4H_9$ | $n-C_4H_9$ | $CH_3$ | $n_D^{40}$ : 1,5053 |
| 13 | $n-C_4H_9$ | $n-C_4H_9$ | $n-C_4H_9$ | $n_D^{40}$ : 1,4971 |
| 14 | $CH_3$ | $CH_3$ | 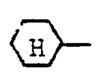 | |

Beispiel 2

Insektizide Frassgift-Wirkung : Spodoptera littoralis, Dysdercus fasciatus und Heliothis virescens

Baumwollpflanzen wurden mit einer wässrigen Emulsion enthaltend 0,05 % der zu prüfenden Verbindung (erhalten aus einem 10 %-igen emulgierbaren Konzentrat) besprüht.

Nach dem Antrocknen des Belages wurden die Pflanzen je mit Larven der Spezies Spodoptera littoralis (L3-Stadium) Dysdercus fasciatus (L4) oder Heliothis virescens (L3) besetzt. Man verwendete pro Versuchsverbindung und pro Test-Spezies zwei Pflanzen und eine Auswertung der erzielten Abtötungsrate erfolgte nach 2, 4, 24 und 48 Stunden. Der Versuch wurde bei 24 °C und 60 % relativer Luftfeuchtigkeit durchgeführt.

Verbindungen der Formel I gemäss Beispiel 1 zeigen im obigen Versuch eine gute Wirkung gegen Larven der Spezies Spodoptera littoralis, Dysdercus fasciatus und Heliothis virescens.

Beispiel 3

Insektizide Frassgift-Wirkung : Leptinotarsa decemlineata

Bei gleicher Arbeitsweise unter Verwendung von Larven der Spezies Leptinotarsa decemlineata (L3) und Kartoffelpflanzen anstelle von Baumwollfplanzen wurde die im Beispiel 2 beschriebene Versuchsmethode wiederholt.

In diesem Versuch ergeben Verbindungen der Formel I eine gute Wirkung gegen Larven der Spezies Leptinotarsa decemlineata.

Beispiel 4

Insektizide Kontakt-Wirkung : Myzus persicae

In Wasser angezogene Pflanzen (Vicia faba) wurden vor dem Versuchsbeginn je mit ca. 200 Individuen der Spezies Myzus persicae besiedelt. Die so behandelten Pflanzen wurden 3 Tage später mit einer Lösung enthaltend 10 oder 1 ppm der zu prüfenden Verbindung aus 30 cm Distanz bis zur Tropfnässe besprüht. Man verwendete pro Test-Verbindung und pro Konzentration zwei Pflanzen, und eine Auswertung der erzielten Abtötungsrate erfolgte nach weiteren 24 Stunden.

Verbindungen der Formel I gemäss Beispiel 1 zeigten im obigen Versuch eine gute Wirkung gegen Insekten der Spezies Myzus persicae.

Beispiel 5

Insektizide systemische Wirkung : Aphis craccivora

Bewurzelte Bohnenpflanzen wurden in Töpfe, welche 600 ccm Erde enthalten, verpflanzt und anschliessend 50 ml einer Lösung der zu prüfenden Verbindung (erhalten aus einem 25 %igen Spritzpulver) in einer Konzentration von 25 ppm, 5 ppm bzw. 1 ppm direkt auf die Erde gegossen.

Nach 24 Stunden wurden auf die oberirdischen Pflanzenteile Blattläuse (Aphis craccivora) gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgte 24 und 48 Stunden nach Versuchsbeginn. Pro Konzentrationsdosis Testsubstanz wurden zwei Pflanzen, je eine in einem separaten Topf, verwendet. Der Versuch wurde bei 25 °C und 70 % relativer Luftfeuchtigkeit durchgeführt.

Die Verbindungen gemäss Beispiel 1 zeigten im obigen Versuch eine positive systemische Wirkung gegen Insekten der Spezies Aphis craccivora.

**Ansprüche** (für die Vertragsstaaten : BE, CH, DE, FR, GB, IT, NL)

1. Eine Verbindung der Formel I

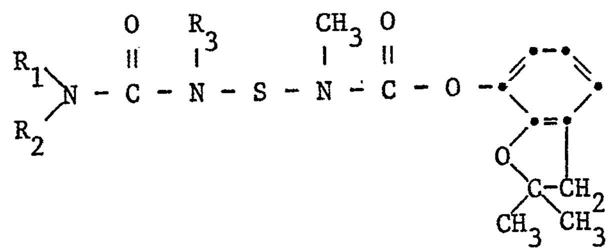

(I)

worin $R_1$ und $R_2$ je eine $C_1$-$C_4$-Alkylgruppe und $R_3$ eine $C_1$-$C_{10}$-Alkyl- oder $C_3$-$C_6$-Cycloalkylgruppe bedeuten.

2. Eine Verbindung gemäss Anspruch 1, worin $R_1$ und $R_2$ je eine Methyl- oder Aethylgruppe und $R_3$ eine $C_1$-$C_8$-Alkyl- oder Cyclopropylgruppe bedeuten.

3. Eine Verbindung gemäss Anspruch 2, worin $R_1$ und $R_2$ je eine Methyl- oder Aethylgruppe und $R_3$ eine $C_1$-$C_4$-Alkyl- oder Cyclopropylgruppe bedeuten.

4. Eine Verbindung gemäss Anspruch 3, worin $R_1$ und $R_2$ je eine Methyl- oder Aethylgruppe und $R_3$ eine Methyl- oder Cyclopropylgruppe bedeuten.

5. Die Verbindung gemäss Anspruch 4 der Formel

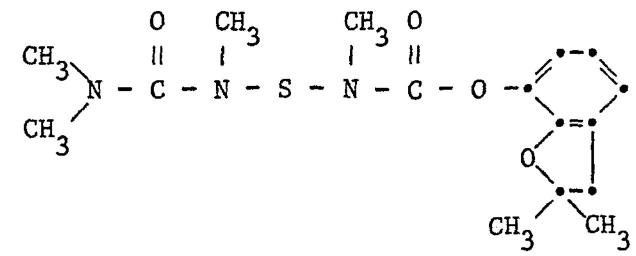

6. Die Verbindung gemäss Anspruch 4 der Formel

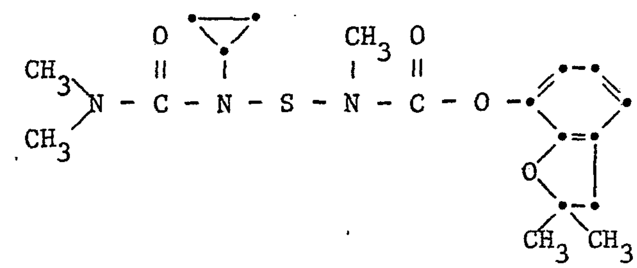

7. Die Verbindung gemäss Anspruch 4 der Formel

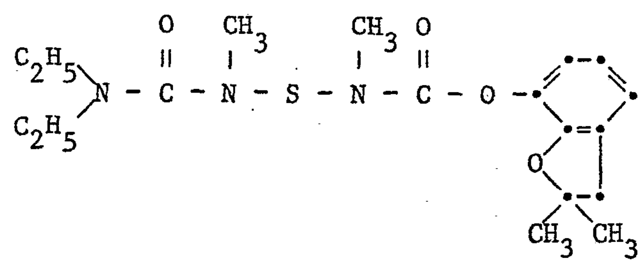

8. Verfahren zur Herstellung von Verbindungen gemäss Anspruch 1, dadurch gekennzeichnet, dass man eine Verbindung der Formel II

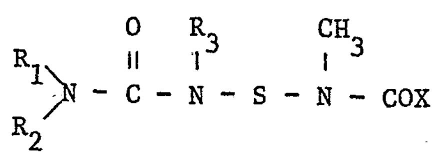

(II)

in Gegenwart einer Base mit der Verbindung der Formel III

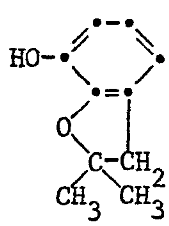

(III)

umsetzt, worin $R_1$, $R_2$ und $R_3$ die im Anspruch 1 angegebene Bedeutung haben und X für ein Halogenatom steht, oder eine Verbindung der Formel IV

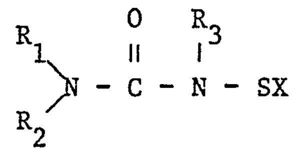

(IV)

in Gegenwart einer Base mit einer Verbindung der Formel V

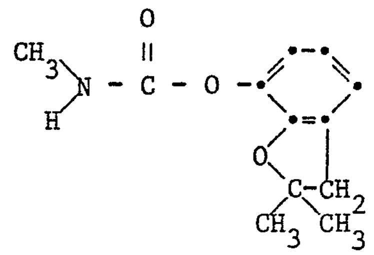

(V)

umzetzt, worin $R_1$, $R_2$, $R_3$ und X die oben angegebene Bedeutung haben.

9. Ein insektizides Mittel, welches als aktive Komponente eine Verbindung gemäss Anspruch 1 und geeignete Träger- oder Zuschlagstoffe oder Gemische solcher Stoffe enthält.

10. Die Verwendung von Verbindungen gemäss Anspruch 1 zur Bekämpfung von Insekten.

**Ansprüche** (für den Vertragsstaat AT)

1. Insektizides Mittel enthaltend als aktive Komponente eine Verbindung der Formel I

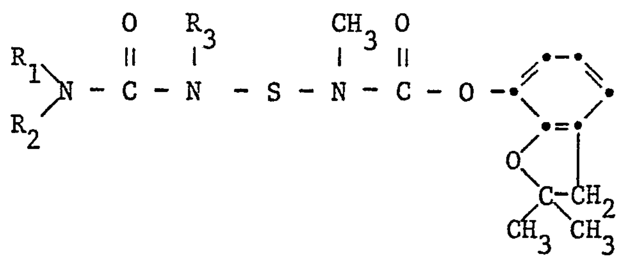

(I)

worin $R_1$ und $R_2$ je eine $C_1$-$C_4$-Alkylgruppe und $R_3$ eine $C_1$-$C_{10}$-Alkyl- oder $C_3$-$C_6$-Cycloalkylgruppe bedeuten.

2. Insektizides Mittel gemäss Anspruch 1 enthaltend als aktive Komponente eine Verbindung der Formel I, worin $R_1$ und $R_2$ je eine Methyl- oder Aethylgruppe und $R_3$ eine $C_1$-$C_8$-Alkyl- oder Cyclopropylgruppe bedeuten.

3. Insektizides Mittel gemäss Anspruch 2, enthaltend als aktive Komponente eine Verbindung der Formel I, worin $R_1$ und $R_2$ je eine Methyl- oder Aethylgruppe und $R_3$ eine $C_1$-$C_4$-Alkyl- oder Cyclopropylgruppe bedeuten.

4. Insektizides Mittel gemäss Anspruch 3, enthaltend als aktive Komponente eine Verbindung der Formel I, worin $R_1$ und $R_2$ je eine Methyl- oder Aethylgruppe und $R_3$ eine Methyl- oder Cyclopropylgruppe bedeuten.

5. Insektizides Mittel gemäss Anspruch 4, enthaltend als aktive Komponente eine Verbindung der Formel

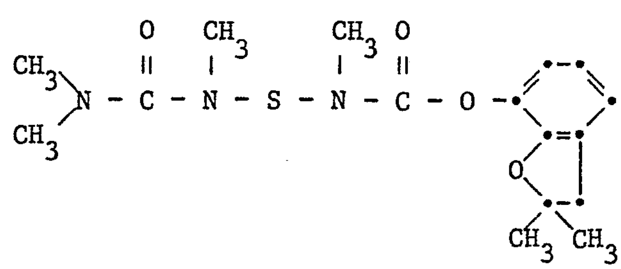

6. Insektizides Mittel gemäss Anspruch 4 enthaltend als aktive Komponente eine Verbindung der Formel

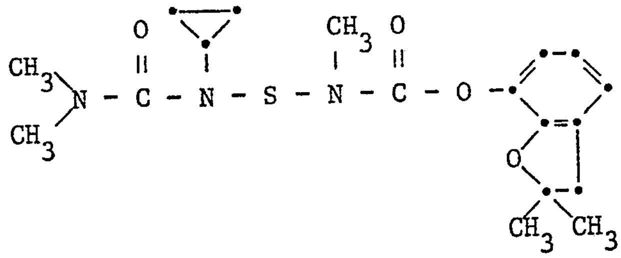

7. Insektizides Mittel gemäss Anspruch 4 enthaltend als aktive Komponente eine Verbindung der Formel

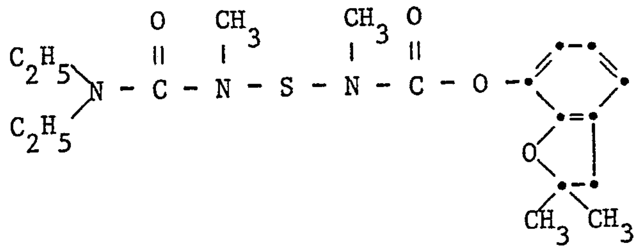

8. Verfahren zur Herstellung von im Anspruch 1 definierten Verbindungen der Formel I, dadurch gekennzeichnet, dass man eine Verbindung der Formel II

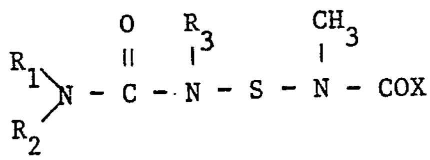

(II)

in Gegenwart einer Base mit einer Verbindung der Formel III

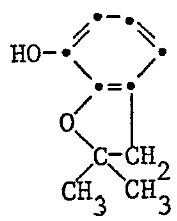

(III)

umsetzt, worin $R_1$, $R_2$ und $R_3$ die entsprechenden im Anspruch 1 angegebenen Bedeutungen haben und X ein Halogenatom bedeutet, oder eine Verbindung der Formel IV

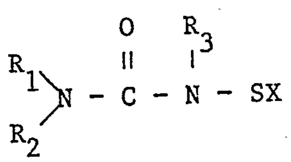

(IV)

in Gegenwart einer Base mit einer Verbindung der Formel V

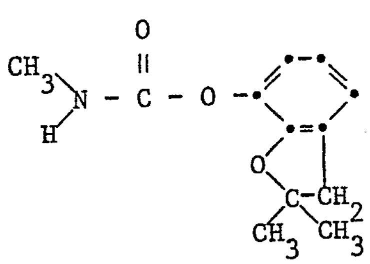

(V)

umsetzt, worin $R_1$, $R_2$, $R_3$ und X die oben angegebenen Bedeutungen haben.

9. Verwendung von einer oder mehreren der im Anspruch 1 definierten Verbindung der Formel I zur Bekämpfung von Insekten.

**Claims** (for the Contracting States : BE, CH, DE, FR, GB, IT, NL)

1. A compound of the formula I

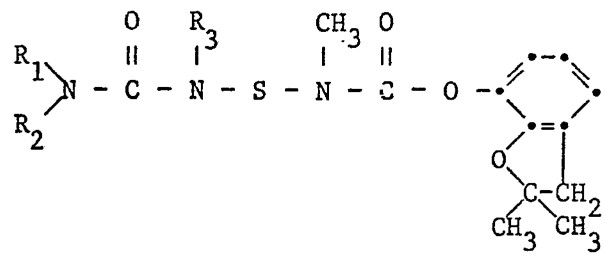

(I)

wherein $R_1$ and $R_2$ are each a $C_1$-$C_4$-alkyl group, and $R_3$ is a $C_1$-$C_{10}$-alkyl or $C_3$-$C_6$-cycloalkyl group.

2. A compound according to claim 1, wherein $R_1$ and $R_2$ are each a methyl or ethyl group, and $R_3$ is a $C_1$-$C_8$-alkyl or cyclopropyl group.

3. A compound according to claim 2, wherein $R_1$ and $R_2$ are each a methyl or ethyl group, and $R_3$ is a $C_1$-$C_4$-alkyl or cyclopropyl group.

4. A compound according to claim 3, wherein $R_1$ and $R_2$ are each a methyl or ethyl group, and $R_3$ is a methyl or cyclopropyl group.

5. The compound according to claim 4 of the formula

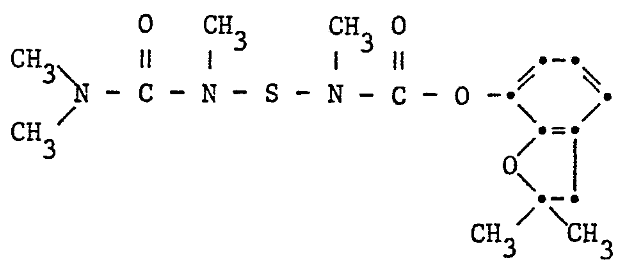

6. The compound according to claim 4 of the formula

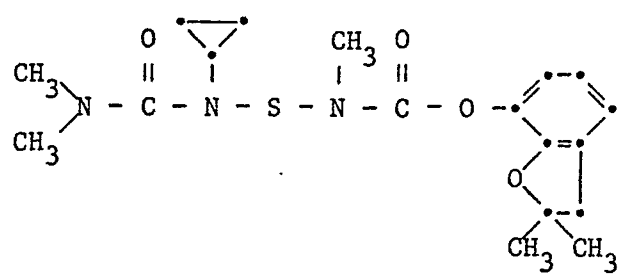

7. The compound according to claim 4 of the formula

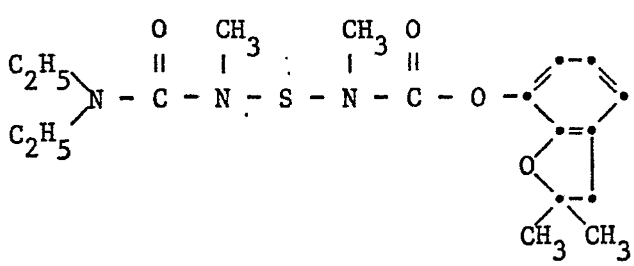

8. Process for producing compounds according to claim 1, characterised in that a compound of the formula II

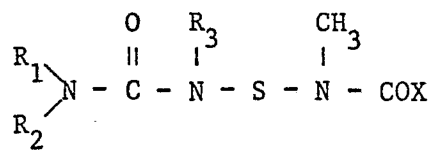

(II)

is reacted, in the presence of a base, with the compound of the formula III

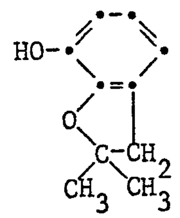

(III)

wherein $R_1$, $R_2$ and $R_3$ have the meanings defined in claim 1, and X is a halogen atom ; or a compound of the formula IV

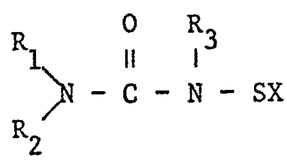

(IV)

is reacted, in the presence of a base, with a compound of the formula V

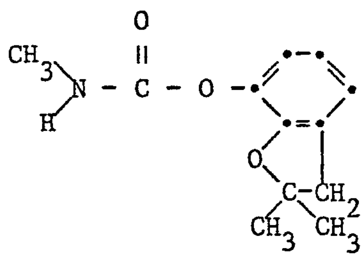

(V)

wherein $R_1$, $R_2$, $R_3$ and X have the meanings given above.

9. An insecticidal composition which contains as active ingredient a compound according to claim 1, and suitable carriers or additives, or mixtures of such substances.

10. The use of compounds according to claim 1 for controlling insects.

**Claims** (for the Contracting State AT)

1. Insecticidal composition containing as active ingredient a compound of the formula I

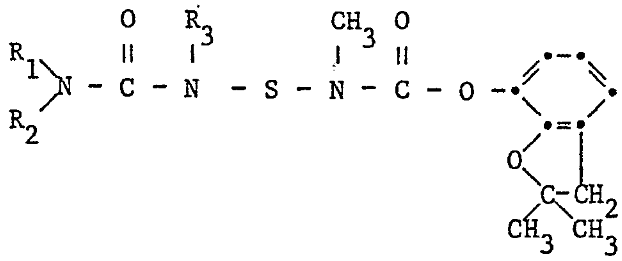

(I)

wherein $R_1$ and $R_2$ are each a $C_1$-$C_4$-alkyl group, and $R_3$ is a $C_1$-$C_{10}$-alkyl or $C_3$-$C_6$-cycloalkyl group.

2. Insecticidal composition according to claim 1, containing as active ingredient a compound of the formula I wherein $R_1$ and $R_2$ are each a methyl or ethyl group, and $R_3$ is a $C_1$-$C_8$-alkyl or cyclopropyl group.

3. Insecticidal composition according to claim 2, containing as active ingredient a compound of the

formula I wherein $R_1$ and $R_2$ are each a methyl or ethyl group, and $R^3$ is a $C_1$-$C_4$-alkyl or cyclopropyl group.

4. Insecticidal composition according to claim 3, containing as active ingredient a compound of the formula I wherein $R_1$ and $R_2$ are each a methyl or ethyl group, and $R_3$ is a methyl or cyclopropyl group.

5. Insecticidal composition according to claim 4, containing as active ingredient a compound of the formula

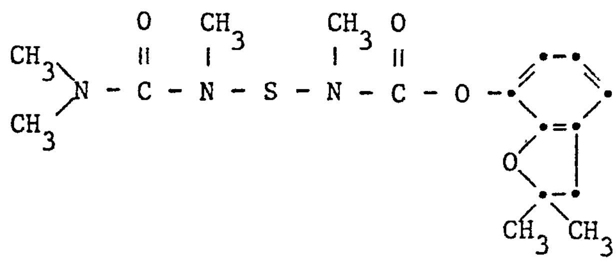

6. Insecticidal composition according to claim 4, containing as active ingredient a compound of the formula

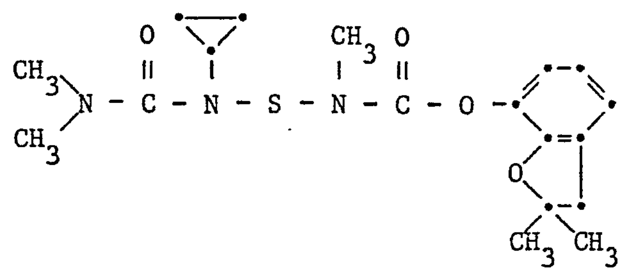

7. Insecticidal composition according to claim 4, containing as active ingredient a compound of the formula

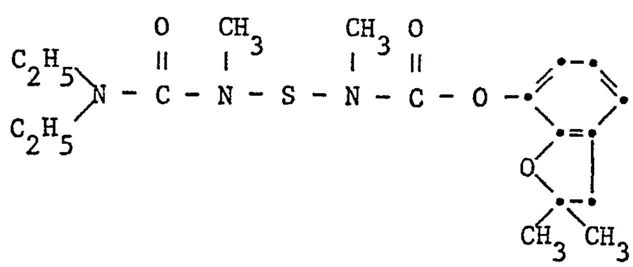

8. Process for producing compounds of the formula I defined in claim 1, characterised in that a compound of the formula II

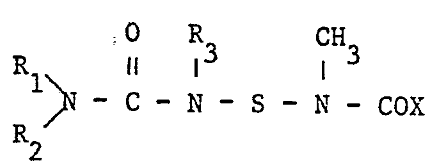

(II)

is reacted, in the presence of a base, with a compound of the formula III

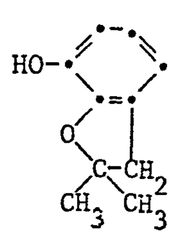

(III)

wherein $R_1$, $R_2$ and $R_3$ have the corresponding meanings given in claim 1, and X is a halogen atom ; or a compound of the formula IV

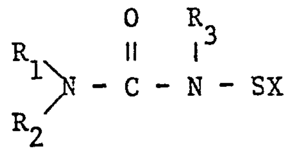

(IV)

is reacted, in the presence of a base, with a compound of the formula V

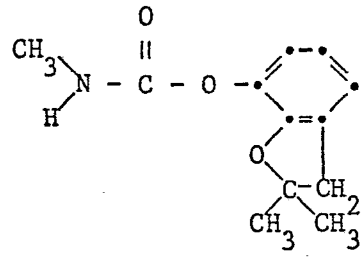

(V)

wherein $R_1$, $R_2$, $R_3$ and X have the meanings given above.

9. Use of one or more of the compounds of the formula I defined in claim 1 for controlling insects.

**Revendications** (pour les Etats contractants : BE, CH, DE, FR, GB, IT, NL)

1. Composé de formule I

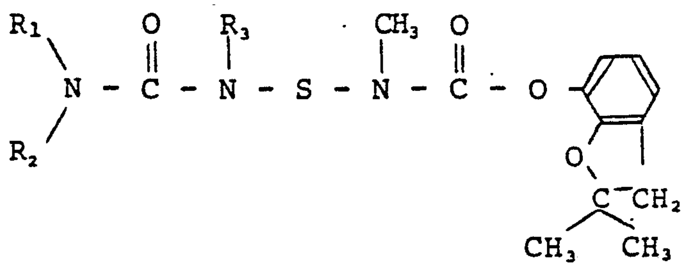

(I)

où $R_1$ et $R_2$ représentent chacun un groupe alcoyle en $C_1$ à $C_4$ et $R_3$ représente un groupe alcoyle en $C_1$ à $C_{10}$ ou cycloalcoyle en $C_3$ à $C_6$.

2. Composé selon la revendication 1, où $R_1$ et $R_2$ représentent chacun un groupe méthyle ou éthyle et $R_3$ représente un groupe alcoyle en $C_1$ à $C_8$ ou cyclopropyle.

3. Composé selon la revendication 2, où $R_1$ et $R_2$ représentent chacun un groupe méthyle ou éthyle et $R_3$ représente un groupe alcoyle en $C_1$ à $C_4$ ou cyclopropyle.

4. Composé selon la revendication 3, où $R_1$ et $R_2$ représentent chacun un groupe méthyle ou éthyle et $R_3$ représente un groupe méthyle ou cyclopropyle.

5. Le composé selon la revendication 4 de formule

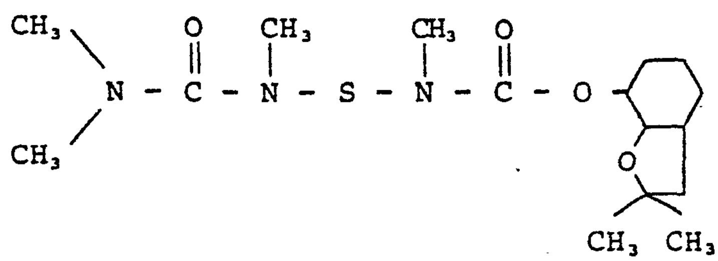

6. Le composé selon la revendication 4 de formule

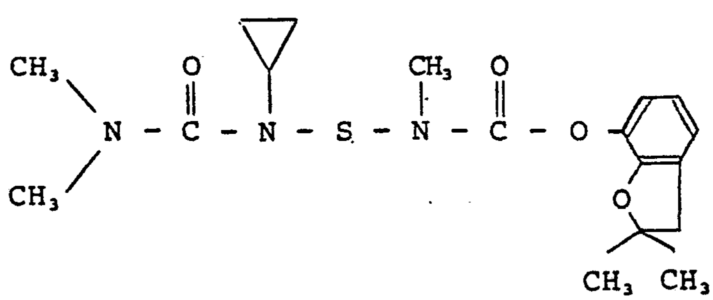

7. Le composé selon la revendication 4 de formule

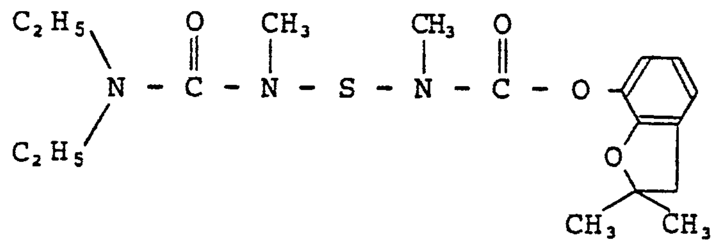

8. Procédé de préparation de composés selon la revendication 1, caractérisé en ce qu'on fait réagir un composé de formule II

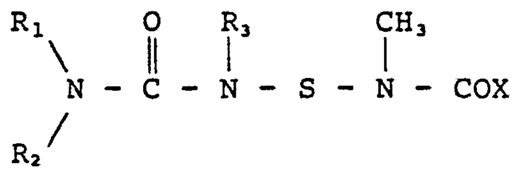

(II)

en présence d'une base avec le composé de formule III

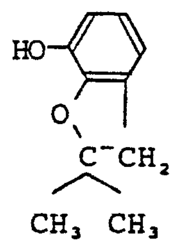

(III)

où $R_1$, $R_2$ et $R_3$ ont la signification donnée dans la revendication 1 et X représente un atome d'halogène, ou un composé de formule IV

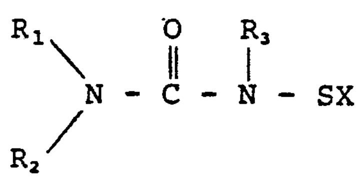

(IV)

en présence d'une base avec un composé de formule V

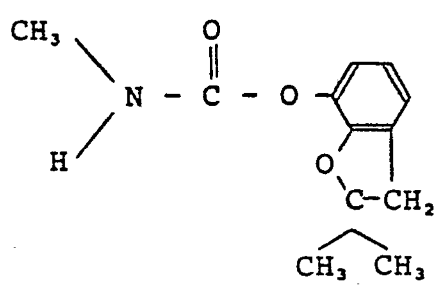

(V)

où $R_1$, $R_2$, $R_3$ et X ont la signification donnée ci-dessus.

9. Agent insecticide contenant comme composant actif un composé selon la revendication 1 et des supports ou additifs appropriés ou des mélanges de tels corps.

10. Application de composés selon la revendication 1 à la lutte contre les insectes.

**Revendications** (pour l'Etat contractant AT)

1. Agent insecticide contenant comme composant actif un composé de formule I

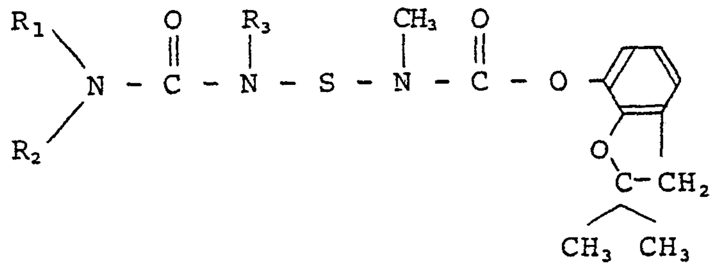

(I)

où $R_1$ et $R_2$ représentent chacun un groupe alcoyle en $C_1$ à $C_4$ et $R_3$ représente un groupe alcoyle en $C_1$ à $C_{10}$ ou un groupe cycloalcoyle en $C_3$ à $C_6$.

2. Agent insecticide selon la revendication 1, contenant comme composant actif un composé de formule I où $R_1$ et $R_2$ représentent chacun un groupe méthyle ou éthyle et $R_3$ représente un groupe alcoyle en $C_1$ à $C_8$ ou cyclopropyle.

3. Agent insecticide selon la revendication 2, contenant comme composant actif un composé de formule I où $R_1$ et $R_2$ représentent chacun un groupe méthyle ou éthyle et $R_3$ représente un groupe alcoyle en $C_1$ à $C_4$ ou cyclopropyle.

4. Agent insecticide selon la revendication 3, contenant comme composant actif un composé de formule I, où $R_1$ et $R_2$ représentent chacun un groupe méthyle ou éthyle et $R_3$ représente un groupe méthyle ou cyclopropyle.

5. Agent insecticide selon la revendication 4, contenant comme composant actif un composé de formule

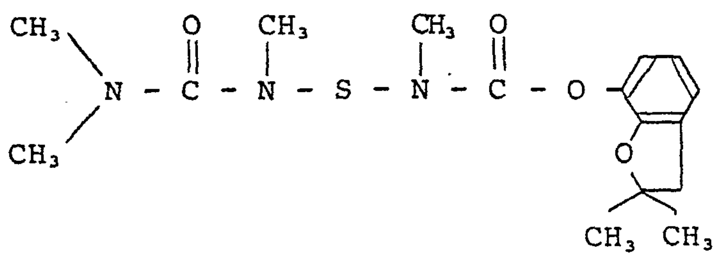

6. Agent insecticide selon la revendication 4 contenant comme composant actif un composé de formule

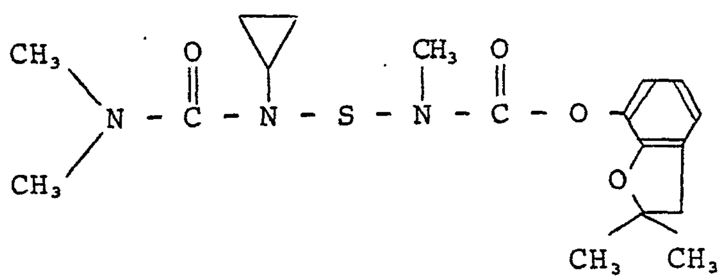

7. Agent insecticide selon la revendication 4 contenant comme composant actif un composé de formule

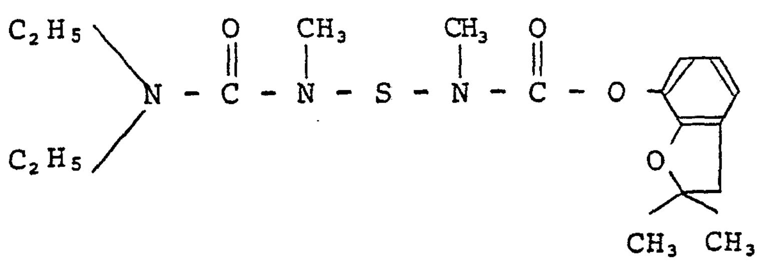

8. Procédé de préparation de composés de formule I définie dans la revendication 1, caractérisé en ce qu'on fait réagir un composé de formule II

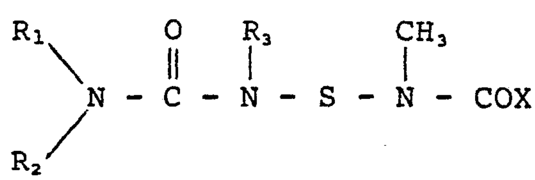

(II)

en présence d'une base avec un composé de formule III

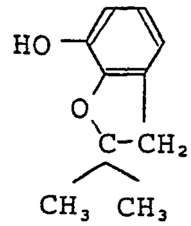

(III)

où $R_1$, $R_2$ et $R_3$ ont les significations correspondantes données dans la revendication 1 et X représente un atome d'halogène, ou en ce qu'on fait réagir un composé de formule IV

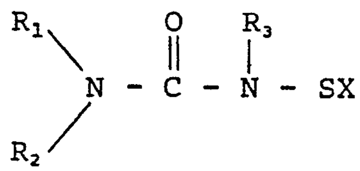

(IV)

en présence d'une base avec un composé de formule V

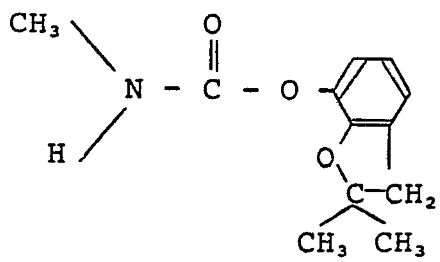

(V)

où $R_1$, $R_2$, $R_3$ et X ont les significations données ci-dessus.

9. Application d'un ou plusieurs des composés de formule I définis dans la revendication 1 à la lutte contre les insectes.